(19) **Europäisches Patentamt**

**European Patent Office**

**Office européen des brevets**

(11) Publication number: **0 188 313 B1**

# EUROPEAN PATENT SPECIFICATION

(45) Date of publication of patent specification: **14.07.93**    (51) Int. Cl.⁵: **A61K 7/18**

(21) Application number: **86300043.6**

(22) Date of filing: **06.01.86**

(54) **Oral hygiene compositions.**

(30) Priority: **14.01.85 GB 8500871**

(43) Date of publication of application:
**23.07.86 Bulletin 86/30**

(45) Publication of the grant of the patent:
**14.07.93 Bulletin 93/28**

(84) Designated Contracting States:
**CH DE FR GB IT LI NL**

(56) References cited:
**US-A- 3 227 617**
**US-A- 3 282 792**

(73) Proprietor: **BEECHAM GROUP plc**
**Four New Horizons Court Harlequin Avenue**
**Brentford Middlesex TW8 9EP(GB)**

(72) Inventor: **Duke, Susan Ann,SmithKline Beecham Consumer Brands**
**St Georg's Avenue**
**Weybridge Surrey KT13 0DE(GB)**
Inventor: **Reading, Julian Peter, SmithKline Beecham**
**St George's Avenue**
**Weybridge, Surrey KT13 0DE(GB)**

(74) Representative: **Connell, Anthony Christopher et al**
**SmithKline Beecham Corporate Patents**
**Great Burgh Yew Tree Bottom Road**
**Epsom, Surrey KT18 5XO (GB)**

**Description**

The present invention relates to oral hygiene compositions, and in particular to dentifices and mouth washes having improved anticaries effect.

A variety of anticaries agents have hitherto been used in dentifrices and mouthwashes, including alkali metal fluorides and alkali metal monofluorophosphates. It has now been discovered that enhanced anticaries effect can be achieved when a combination of fluoride ions and hydrogen citrate ions is used within a specified weight ratio and below a certain pH threshold.

In the past, oral hygiene compositions comprising a fluoride ion source and citrate have been disclosed. Thus, US 3 282 792 discloses compositions comprising stannous fluoride and a hydroxyl substituted aliphatic acid such as citric acid, the function of the latter being to maintain stannous ions in solution, to avoid formation of an insoluble precipitate. Us 3 227 617 discloses dentifrices comprising, as an abrasive, a mixture of an insoluble alkali metal metaphosphate and an insoluble calcium or magnesium polishing agent which is compatible with fluoride. Citric acid may be added as a buffer, to provide dentifrices with pHs in the range 5 to 6.5. EP 0 095 871 discloses oral hygiene compositions having a combination of potassium ions and citrate ions, for use as an antisensitivity system. A fluoride ion source may be added as an optional extra. In each case however there is no suggestion that hydrogen citrate may enhance the anticaries effect of fluoride ions.

With reference to German patent application No. 3 526 654 (Lion Corporation), the applicant has voluntarily limited the scope of the present application and submitted separate claims for Germany.

Accordingly the present invention provides for the use of hydrogen citrate ions and a source of fluoride ions in a weight ratio of from 10:1 to 500:1, for the manufacture of a mouthwash or a dentifrice for anti-caries therapy, such that in the mouthwash the concentration of hydrogen citrate ions is from $1 \times 10^{-3}$ to $1 \times 10^{-1}$M and in the dentifrice the concentration of hydrogen citrate ions is from $4 \times 10^{-3}$ to $4 \times 10^{-1}$M, with the fluoride ions provided by an alkali metal monofluorophosphate and/or an alkali metal fluoride, and the mouthwash or dentifrice having a pH of less than 7.

The preferred weight ratio of hydrogen citrate ions to fluoride ions is from 50:1 to 250:1

Preferably, the hydrogen citrate ions are provided by a soluble alkali metal salt, such as disodium hydrogen citrate.

Preferably, the fluoride ions are provided by an alkali metal monofluorophosphate and/or an alkali metal fluoride.

Preferably, the concentration of hydrogen citrate ions is from $5 \times 10^{-3}$M to $3 \times 10^{-2}$M in a mouthwash, and from $2 \times 10^{-2}$M to $1.2 \times 10^{-1}$M in a dentifrice.

Preferably, the pH of the composition is from 4.0 to 5.0.

The percentage by weight of citrate material in the compositions of the invention will, of course, depend on the molecular weight of the material.

However, in general, a dentifrice composition of the invention will contain from 0.1% to 10% % by weight of alkali metal hydrogen citrate, preferably from 0.4% to 4.0%, and a mouthwash will contain from 0.025% to 2.5% by weight of alkali metal hydrogen citrate, preferably from 0.1% to 1.0% by weight of the composition.

The preferrred alkali metal fluoride is sodium fluoride, and the preferred alkali metal monofluorophosphate is sodium monofluorophosphate. The composition of the invention preferably comprises from 100 ppm to 2500 ppm of fluoride ions in total. When the composition is a dentifrice, preferably from 10 to 90%, more preferably 40 to 80%, of the fluoride is provided by the alkali metal fluoride and the remainder is provided by the alkali metal monofluorophosphate. When the composition is a mouthwash, the fluoride may be provided entirely by alkali metal fluoride.

A dentifrice composition according to the invention preferably includes a dentally acceptable abrasive in the orally acceptable carrier, and the choice of abrasive need only be limited by the necessity of keeping the pH of the final composition below 7. Calcium carbonate has been found to give a composition pH above 7, so is not suitable for use in the present invention.

Examples of suitable abrasives are dicalcium phosphate, alumina, calcium pyrophosphate, pumice, methyl methacrylate and silica, the latter being particularly preferred.

The silica abrasive can be a precipitated silica or a silica gel, such as the silica xerogels described in US Patent No. 3538230. Preferred silica xerogels are marketed under the trade name 'Syloid' by W.R. Grace and Company, Davison Chemical Division. Preferred precipitated silicas are those marketed under the tradename 'Zeodent' by the J.M. Huber Corporation.

The amount of abrasive will be within conventional limits, and will normally vary from about 15% to 50% by weight of the composition.

The compositions of the invention may optionally contain other agents known to enhance the anticaries effect of fluoride and monofluorphosphate, such as calcium glycerophosphate, this being incorporated in a weight ratio of up to 1:3, preferably 1:20 to 1:3, to the total weight of fluoride and/or monofluorophosphate salts.

The composition of the invention may be presented in conventional toothpaste, dental cream and dental powder formulations, or as a conventional mouthwash formulation.

Compositions according to the invention are able to reduce plaque growth and increase plaque pH thereby reducing acid formation and hence reducing the severity of attack on the tooth enamel. The increase in plaque pH is significantly enhanced by hydrogen citrate ions, as shown in the data section below.

Compositions according to the present invention may be produced by admixture of the various ingredients and may contain the usual optional accessory ingredients such as detergents, dyes, sweetening agents, for example soluble saccharin, flavouring oils, for example oils of spearmint, wintergreen or peppermint, chloroform, colouring or whitening agents for example titanium dioxide, preservatives for example sodium benzoate, emulsifying agents, silicones, alcohol, menthol chlorophyll compounds for example sodium copper chlorophyllin, antibacterial agents, for example chlorhexidine, anti-plaque agents, anti-calculus agents, agents for sensitive dentine for example strontium salts or formaldehyde, and agents which enhance the anticaries activity of fluorides (e.g. calcium glycerophosphate).

The compositions of the invention may, accordingly, be prepared by admixing hydrogen citrate and fluoride ion sources in an aqueous medium, and combining the resultant aqueous solution with an orally acceptable carrier at a temperature below the boiling point of water to produce a composition with a pH less than 7.

In another aspect, the invention also provides a use in which the composition is a dentifrice and the abrasive is silica.

In a further aspect, the invention provides a dentifrice in which the abrasive is selected from dicalcium phosphate, alumina, calcium pyrophosphate, pumice, methyl methacrylate and silica, and excluding dentifrices comprising dipotassium citrate and methyl methacrylate.

The invention is illustrated by the following examples.

Example 1

| Mouthwash formula | | |
|---|---|---|
| | | % w/w |
| 70% sorbitol | | 5.00 |
| Saccharin | | 0.003 |
| *Nipasept | | 0.1 |
| **Bromopol | | 0.01 |
| Dyes | (Blue No. 1) | 0.001 |
| | (Yellow No. 10) | 0.003 |
| Flavours | | 0.05 |
| Sodium fluoride | | 0.032 |
| Sodium monofluorophosphate | | 0.1 |
| Disodium hydrogen citrate | | 0.40 |
| Water to 100% | | |

\* Nipasept is a Trade Mark of Nipa Labs.
Mixture of sodium salts - Methylhydroxybenzoate
Ethylhydroxybenzoate
Propylhydroxybenzoate
\*\* Bromopol is a Trade Mark of the Boots Company.
2 bromo-2-nitropane-1, 3-diol

Example 2

| Toothpaste in silica Base | |
|---|---|
| | % w/w |
| *Zeodent 113 | 18.00 |
| Sorbitol | 50.00 |
| ** Syloblanc 34 | 3.50 |
| Glycerin | 4.50 |
| Carboxymethyl cellulose CMC 7MF | 1.00 |
| Flavour | 0.60 |
| 15% Saccharin solution | 1.20 |
| Sodium monofluorophosphate | 0.38 |
| Sodium fluoride | 0.11 |
| Disodium hydrogen citrate | 1.60 |
| Nipagin Sodium | 0.15 |
| *** 30% Empicol | 4.00 |
| Calcium glycerophosphate | 0.07 |
| Water | 13.24 |
| **** Natrosol 250H | 1.50 |
| 0.1% FD $_\epsilon$ C No 1 Dye | 0.15 |

*Zeodent 113 is a Trade Mark of Huber Corporation.
Precipitated Silica.
**Syloblanc 34 is a Trade Mark of Grace and Co.
Precipitated Silica.
***Empicol is a Trade Mark of Allbright & Wilson.
Sodium lauryl sulphate.
****Natrosol 250H is a Trade Mark of Hercules Ltd.
Hydroxyethyl cellulose gum.

Example 3

| Gel Formula | | |
|---|---|---|
| | Translucent %w/w | Clear %w/w |
| 70% Sorbitol Solution | 50.00 | 65.00 |
| PEG 300 | 3.00 | 3.00 |
| *Tixosil 53 BE | 16.00 | 16.00 |
| **Aerosil 200 | 2.50 | 2.20 |
| ***CMC 7M8 5XF | 1.00 | 0.80 |
| 30% Sodium lauryl Sulphate | 7.50 | 7.50 |
| ****PVP K29-32 | 0.10 | 0.10 |
| Butylated hydroxy toluene | 0.10 | 0.10 |
| Disodium hydrogen citrate. | 0.60 | 0.60 |
| Sodium monofluorophosphate | 0.80 | 0.80 |
| Calcium glycerophosphate | 0.13 | 0.13 |
| Flavour | 0.90 | 0.90 |
| Saccharin | 0.20 | 0.20 |
| 0.2% F.D + C Blue No. 1 | 0.875 | 0.875 |
| Water | 16.295 | 1.795 |

*Tixosil 53 BE is a Trade Mark of Rhone-Poulenc
Silica.
** Aerosil 200 is a Trade Mark of Degussa.
Silica.
*** CMC 7M8 5XF is a Trade Mark of Hercules Ltd.
Carboxymethyl cellulose.
**** PVP K29-32 is a Trade Mark of G.A.S. Corporation.
Polyvinyl pyrollidone.

Test Data

1. Effect on the pH of dental plaque

20 subjects were randomly allocated to a treatment and were allowed to use a test mouthwash or toothpaste for 1 week ad.lib. They then refrained from all oral hygiene measures for 24 hours. On the test day each subject refrained from eating or drinking for 1 hour prior to and during the test period. Each subject rinsed his mouth with 10 ml of a 3:1 slurry in water of the test toothpaste, or the test mouthwash. 30 minutes later plaque samples were collected from the molars and premolars and the mouth was then rinsed with 10ml of 10% w/v aqueous sucrose.

Ten minutes later plaque samples were again taken from the molars and premolars.

Each plaque sample, immediately following sampling, was suspended in $20\mu l$ physiological saline and the pH was measured using a microcombination electrode.

The whole procedure was repeated on two further occasions.

The results are presented in the Table.

### Table I

| Composition | Plaque pH after aqueous sucrose treatment |
| --- | --- |
| 1. Mouthwash containing 0.055% NaF 0.031% disodium hydrogen citrate (initial pH 5.0) | $6.15 \pm 0.20$ |
| 2. Mouthwash containing 0.055% NaF (initial pH 5.0) | $5.94 \pm 0.21$ |

## Table II

| Composition | Plaque pH after aqueous sucrose treatment |
|---|---|
| Dentifrice containing<br><br>1.  0.11% NaF, 0.38% $Na_2PO_3F$<br>    0.30% disodium hydrogen citrate<br>    Precipitated silica (Zeodent 113) abrasive (initial pH 5.0) | $6.04 \pm 0.24$ |
| Dentifrice containing<br><br>2.  0.11% NaF, 0.38% $Na_2PO_3F$<br>    Precipitated silica (zeodent 113) abrasive (initial pH 5.0) | $5.80 \pm 0.21$ |

The results presented in Tables I and II show that the addition of disodium hydrogen citrate to both fluoride mouthwashes and dentifrices significantly reduced the plaque pH fall following a challenge with aqueous sucrose over and above that from the fluoride formulation alone. Therefore the addition of disodium hydrogen citrate to such formulations would appear to improve the ability of the formulation to reduce dental caries.

**Claims**

1. The use of hydrogen citrate ions and a source of fluoride ions in a weight ratio of from 10:1 to 500:1, for the manufacture of a mouthwash or a dentifrice for anticaries therapy, such that in the mouthwash the concentration of hydrogen citrate ions is from $1 \times 10^{-3}$ to $1 \times 10^{-1}$M and in the dentifrice the concentration of hydrogen citrate ions is from $4 \times 10^{-3}$ to $4 \times 10^{-1}$M, with the fluoride ions provided by an alkali metal monofluorophosphate and/or an alkali metal fluoride, and the mouthwash or dentifrice having a pH of less than 7.

2. A use according to claim 1, in which hydrogen citrate ions are present in a concentration of from $5 \times 10^{-3}$M to $3 \times 10^{-2}$M in a mouthwash and from $2 \times 10^{-2}$M to $1.2 \times 10^{-1}$M in a dentifrice.

3. A use according to claim 1 or 2, in which the weight ratio of hydrogen citrate ions to fluoride ions is from 50:1 to 250:1.

4. A use according to any one of claims 1 to 3, in which the source of hydrogen citrate ions comprises an alkali metal hydrogen citrate.

**5.** A use according to claim 4, in which the percentage weight of alkali metal hydrogen citrate is from 0.1% to 10%, by weight of the composition when in the form of a dentifrice; or from 0.025% to 2.5% by weight of the composition when in the form of a mouthwash.

**6.** A use according to claim 4 or 5 in which the alkali metal hydrogen citrate salt is disodium hydrogen citrate.

**7.** A use according to any of claims 1 to 6 in which from 100 to 2500 ppm of fluoride ions are present in total.

**8.** A use according to any of claims 1 to 7 in which the pH of the composition is from 4.0 to 5.0.

**9.** A use according to any one of claims 1 to 8 in which the composition is a dentifrice and the abrasive is silica.

**10.** A dentifrice comprising a source of fluoride ions provided by an alkali metal monofluorophosphate and/or an alkali metal fluoride; an orally acceptable carrier and an abrasive characterised in that the composition further comprises hydrogen citrate ions, present in a weight ratio of from 10:1 to 500:1 with respect the fluoride ions and in a concentration of from $4 \times 10^{-3}$ to $4 \times 10^{-1}$M; the composition has a pH of less than 7; the abrasive is selected from dicalcium phosphate, alumina, calcium pyrophosphate, pumice, methyl methacrylate and silica, and excluding dentifrices comprising dipotassium citrate and methyl methacrylate.

**11.** A mouthwash comprising a source of fluoride ions provided by an alkali metal monofluorophosphate and/or an alkali metal fluoride; an orally acceptable carrier and characterised in that the composition further comprises hydrogen citrate ions present in weight ratio of from 10:1 to 500:1 with respect the fluoride ions and in a concentration of from $1 \times 10^{-3}$ to $1 \times 10^{-1}$M; and the composition has a pH of less than 7.

**Patentansprüche**

**1.** Verwendung Von Hydrogencitrationen und einer Quelle von Fluoridionen in einem Gewichtsverhältnis von 10:1 bis 500:1 zur Herstellung von Mundwässern oder Zahnpflegemitteln zur Antikaries-Therapie, wobei die Konzentration von Hydrogencitrationen in dem Mundwasser $1 \times 10^{-3}$ bis $1 \times 10^{-1}$ M ist und im Zahnpflegemittel die Konzentration der Hydrogencitrationen $4 \times 10^{-3}$ bis $4 \times 10^{-1}$M ist, und wobei ein Alkalimetallmonofluorphosphat und/oder Alkalimetallfluorid als Fluoridionenquelle dient, und das Mundwasser oder Zahnpflegemittel einen pH-Wert kleiner als 7 hat.

**2.** Verwendung nach Anspruch 1, wobei die Hydrogencitrationen in dem Mundwasser in einer Konzentration von $5 \times 10^{-3}$ M bis $3 \times 10^{-2}$M und in dem Zahnpflegemittel in einer Konzentration von $2 \times 10^{-2}$ M bis $1.2 \times 10^{-1}$ M vorliegen.

**3.** Verwendung nach Anspruch 1 oder 2, wobei das Gewichtsverhältnis der Hydrogencitrationen zu den Fluoridionen 50:1 bis 250:1 beträgt.

**4.** Verwendung nach einem der Ansprüche 1 bis 3, wobei die Hydrogencitrationenquelle ein Alkalimetallhydrogencitrat umfaßt.

**5.** Verwendung nach Anspruch 4, wobei der prozentuale Gewichtsanteil des Alkalimetallhydrogencitrats im Fall des Zahnpflegemittels 0.1% bis 10%, bzw. im Fall des Mundwassers 0.025% bis 2.5% beträgt.

**6.** Verwendung nach Anspruch 4 oder 5, wobei das Alkalimetallhydrogencitratsalz das Dinatriumhydrogencitrat ist.

**7.** Verwendung nach einem der Anspüche 1 bis 6, wobei Fluoridionen insgesamt in einer Menge von 100 bis 2500 ppm vorliegen.

9

**8.** Verwendung nach einem der Ansprüche 1 bis 7, wobei der pH-Wert der Zusammensetzung bei 4,0 bis 5,0 liegt.

**9.** Verwendung nach einem der Ansprüche 1 bis 8, wobei die Zusammensetzung ein Zahnpflegemittel und das Schleifmittel Siliciumdioxid ist.

**10.** Zahnpflegemittel mit einem Alkalimetallmonofluorphosphat und/oder Alkalimetallfluorid als Fluoridionenquelle, einem im Mund verträglichen Träger und einem Schleifmittel, dadurch gekennzeichnet, daß die Zusammensetzung außerdem Hydrogencitrationen in einer Konzentration von $4\times10^{-3}$ bis $4\times10^{-1}$ M enthält, das Gewichtsverhältnis von Hydrogencitrationen zu Fluoridionen 10:1 bis 500:1 beträgt und die Zusammensetzung einen pH-Wert kleiner als 7 hat, und das Schleifmittel ausgewählt ist aus Dicalciumphosphat, Aluminiumoxid, Calciumpyrophosphat, Bimsstein, Methylmethacrylat oder Siliciumdioxid, ausgenommen Zahnpflegemittel mit einem Gehalt an Dikaliumcitrat und Methylmethacrylat.

**11.** Mundwasser mit einem Alkalimetallmonofluorphosphat und/oder Alkalimetallfluorid als Fluoridionenquelle, einem im Mund verträglichen Träger, dadurch gekennzeichnet, daß die Zusammensetzung außerdem Hydrogencitrationen in einer Konzentration von $1\times10^{-3}$ bis $1\times10^{-1}$M enthält, das Gewichtsverhältnis von Hydrogencitrationen zu Fluoridionen 10:1 bis 500:1 beträgt und die Zusammensetzung einen pH-Wert kleiner als 7 hat.

## Revendications

**1.** Utilisation d'ions hydrogénocitrate et d'une source d'ions fluorure selon un rapport pondéral de 10:1 à 500:1, pour la fabrication d'un collutoire ou d'un dentifrice pour la lutte contre les caries, telle que dans le collutoire, la concentration des ions hydrogénocitrate soit de $1\times10^{-3}$ à $1\times10^{-1}$ M et dans le dentifrice, la concentration des ions hydrogénocitrate soit de $4\times10^{-3}$ à $4\times10^{-1}$ M, les ions fluorure étant fournis par un monofluorophosphate de métal alcalin et/ou un fluorure de métal alcalin, et le collutoire ou le dentifrice ayant un pH inférieur à 7.

**2.** Utilisation suivant la revendication 1, dans laquelle les ions hydrogénocitrate sont présents en une concentration de $5\times10^{-3}$ à $3\times10^{-2}$ M dans un collutoire et de $2\times10^{-2}$ à $1,2\times10^{-1}$ M dans un dentifrice.

**3.** Utilisation suivant les revendications 1 ou 2, dans laquelle le rapport pondéral des ions hydrogénocitrate aux ions fluorure est de 50:1 à 250:1.

**4.** Utilisation suivant l'une quelconque des revendications 1 à 3, dans laquelle la source d'ions hydrogénocitrate comprend un hydrogénocitrate de métal alcalin.

**5.** Utilisation suivant la revendication 4, dans laquelle le pourcentage en poids de l'hydrogénocitrate de métal alcalin est de 0,1% à 10% en poids de la composition lorsque celle-ci est sous la forme d'un dentifrice; ou de 0,025% à 2,5% en poids de la composition lorsqu'elle est sous forme d'un collutoire.

**6.** Utilisation suivant les revendications 4 ou 5, dans laquelle l'hydrogénocitrate de métal alcalin est l'hydrogénocitrate disodique.

**7.** Utilisation suivant l'une quelconque des revendications 1 à 6, dans laquelle la quantité totale présente d'ions fluorure est de 100 à 2500 ppm.

**8.** Utilisation suivant l'une quelconque des revendications 1 à 7, dans laquelle le pH de la composition est de 4,0 à 5,0.

**9.** Utilisation suivant l'une quelconque des revendications 1 à 8, dans laquelle la composition est un dentifrice et l'abrasif est de la silice.

**10.** Dentifrice comprenant une source d'ions fluorure fournis par un monofluorophosphate de métal alcalin et/ou un fluorure de métal alcalin, un support acceptable par voie orale et un abrasif, caractérisé en ce que la composition comprend de plus des ions hydrogénocitrate présents selon un rapport pondéral de 10:1 à 500:1 par rapport aux ions fluorure et à une concentration de $4\times10^{-3}$ à $4\times10^{-1}$ M; la composition

a un pH inférieur à 7; l'abrasif est choisi parmi le phosphate dicalcique, l'alumine, le pyrophosphate de calcium, la pierre ponce, le méthacrylate de méthyle et la silice, et à l'exclusion des dentifrices comprenant du citrate dipotassique et du méthacrylate de méthyle.

11. Collutoire comprenant une source d'ions fluorure fournis par un monofluorophosphate de métal alcalin et/ou un fluorure de métal alcalin, un support acceptable par voie orale, et caractérisé en ce que la composition comprend de plus des ions hydrogénocitrate présents selon un rapport pondéral de 10:1 à 500:1 par rapport aux ions fluorure et à une concentration de $1 \times 10^{-3}$ à $1 \times 10^{-1}$ M, et la composition a pH inférieur à 7.